# EUROPEAN PATENT SPECIFICATION

(11) **EP 2 419 005 B1**
(45) Date of publication and mention of the grant of the patent: **19.11.2014**
(21) Application number: 10717261.1
(22) Date of filing: 12.04.2010
(51) Int. Cl.: A61B 5/00

(54) **NEEDLE WITH INTEGRATED FIBERS IN THE CUTTING FACETS OF THE BEVEL**
NADEL MIT INTEGRIERTEN FASERN IN DEN SCHNEIDFACETTEN DER SCHRÄGKANTE
AIGUILLE AVEC DES FIBRES INTÉGRÉES DANS LES FACETTES COUPANTES DU BISEAU

(30) Priority: 15.04.2009 EP 09157976
(43) Date of publication of application: 22.02.2012
(73) Proprietor: Koninklijke Philips N.V., 5656 AE Eindhoven (NL)
(72) Inventor: HENDRIKS, Bernardus, NL-5656 AE Eindhoven (NL); BRAUN, Augustinus, NL-5656 AE Eindhoven (NL); HARBERS, Rik, NL-5656 AE Eindhoven (NL); VAN DER VOORT, Marjolein, NL-5656 AE Eindhoven (NL); DESJARDINS, Adrien, NL-5656 AE Eindhoven (NL); NACHABE, Rami, NL-5656 AE Eindhoven (NL)
(74) Representative: van Velzen, Maaike Mathilde
(86) International application number: PCT/IB2010/051570
(87) International publication number: WO 2010/119392

(56) References cited:
- WO-A2-2008/045316
- US-A- 5 460 182
- US-A- 6 011 889
- US-A1- 2003 010 396

## Description

### FIELD OF THE INVENTION

The invention generally relates to a needle with integrated fibers. Particularly, the invention relates to a small diameter needle for tissue inspection based on optical spectroscopy, for example to diagnose whether tissue is cancerous or not.

The invention further relates to a system for optical tissue inspection comprising such a needle.

### TECHNOLOGICAL BACKGROUND

Needle interventions are widely used in the field of oncology for taking biopsies of tissue in order to inspect whether tissue is cancerous or not. To make these interventions more reliable, feedback of what kind of tissue is in front of the needle is required. A way to achieve this is by making use of optical spectroscopy. This requires integration of fibers into the needle. These fibers are used to deliver light to illuminate the tissue in front of the needle and to collect back the reflected light from the tissue.

However, a problem with this is how to integrate these fibers into the needle without compromising the original functionality of the needle, hence altering the outer part of the needle or the inner part (for instance used to take a tissue sample). This means that the fibers must be integrated inside the needle wall while still being able to construct different fibers arrangements at the distal end of the needle. This is of particular importance, because the fiber arrangement at the tip of the needle determines the sensitivity in the measurement of certain optical properties. For instance the fact that a fiber tip is slanted or the distance between two fibers at the tip, affects the measured signal.

An important feature in that aspect is the volume of the tissue probed in front of the needle bevel. The probed volume is typically banana-shaped and depends on the distance of the source-detector fibers, on the shape of the fiber exits and on the orientation of the fiber. A problem arising when integrating the fiber distal ends into the bevel of the needle is that extruding parts or indentations are not allowed because this could affect the trajectory of the needle when inserted in the tissue, may damage tissue due to the extruding parts or may lead to unwanted tissue collection in the indented parts. As a result shaping the fiber distal ends and orientation is not possible, limiting the possibilities of fiber end geometries and thus limiting the probing volumes in front of the needle.

The problem is how to integrate the source-detector fibers into the needle bevel without producing indentations or extruding parts on the bevel, while still having freedom in designing the probing volume in front of the needle.

Furthermore, needles employing optical fibers are particular suited to provide physiological information of the tissue in front of the needle. Such a needle containing fibers require that these fibers are connected to an optical console. At the console, light may be coupled into these fibers to illuminate the tissue in front of the needle, while light, being backscattered from the tissue and coupled back into the fiber, may be detected in the console and further processed. This means that the needle may be connected to the console by the fiber.

US 5,460,182 shows and describes trocar tissue penetrating devices with optical fibers, wherein the end faces of the fibers are flush with the trocar facets. Furthermore, a hollow needle is shown and described, with two optical fibers being secured along the minor axis of an ellipsoid plane of a bevel of the needle.

### SUMMARY OF THE INVENTION

It is an object of the invention to provide a needle having at least one fiber exit at the distal end of the needle. It is another object of the invention, to provide a needle which can be used reliably, which will be easily handled, and which can be appropriately sterilized.

It is another object of the invention to provide a system for using the needle.

These and other objects are achieved by the subject matter according to the independent claim. Further embodiments of the present invention are described in the respective dependent claims.

First of all, geometrical aspects will be defined for a better understanding. The needle includes a longitudinal main axis, usually the centre axis of a rotationally symetrical shaft. Further, the tip portion of the needle is cut at an angle to the main axis forming a bevel. Looking onto the slanted surface of the bevel as well as on the shaft means looking from 'above'. Accordingly, 'under' the needle is opposite to 'above'. The pointed tip of the bevel is directed to the 'front' of the needle. As a result, looking from the 'side', it is possible to recognize the angle between the bevel and the main axis.

A main aspect of the invention is the tip of the needle, which comprises a bevel and facets. A 'facet' may be a small and plane surface. Usually, a 'facet' may be realized by cutting away a small area of a body thereby achieving a surface with edges to other surfaces of the body. The contour of a facet may be affected by the angle of cutting. Furthermore, the surface of a facet may be convex or concave, i.e. the facet may be curved forming a part-cylindrical shape. The edges of the facet may preferably be sharpened or may be rounded and thus blunt.

Principally, it is possible to introduce a needle or instrument into tissue by cutting the tissue or displacing the tissue. Accordingly, the edges of a needle or instrument will be sharp or blunt. It will be understood that a combination of cutting and displacing or squeesing the tissue is also possible. Depending from the application, the needle or instrument will more or less cut and/or displace.

According to the invention, the facets together with the bevel may realize a combination of displacing and cutting. The facets provide some areas at which the tissue being in contact with the facet, may not be cut but only squeezed to the side. On the other hand, an edge (especially if sharpened) will provide for a cut of the tissue. Therefore, the tip portion of the needle according to the invention may have a plurality of edges cutting the tissue, and also facet surfaces displacing the tissue while the needle is introduced into the tissue. It may be seen as an advantage that a plurality of smaller cuts together with a certain amount of squeezing will facilitate the introducing of the needle and will cause less pain.

With 'bevel' is meant a geometrical structure, wherein a shaft of a needle includes a circular cross section, and the distal end of a needle shaft, in particular of a shaft of a hollow needle, is cut such that an oval surface is formed, which is inclined relative to the longitudinal axis of the shaft. Further, there is defined an angle between the longitudinal axis of the shaft and the inclined surface, i.e. the bevel.

The wording 'bevel' might also include similar structures at the tip of the needle, which structures are useful for introducing the needle into a tissue. For example, the bevel might be a convex or concave surface, or the bevel might be a combination of several small surfaces, wherein these surfaces are connected to each other by steps or edges. It might also be possible that the cross section of the shaft is not completely cut by the bevel, such that an area remains which is blunt, i.e. is perpendicularly or in another blunt angle orientated relative to the longitudinal axis of the shaft. Such a blunt end might include rounded edges or might also form a rounded leading edge.

It should be noted that the bevel might form an acute angle with the shaft, such that the needle includes a pointed tip. Preferably, the acute angle might be approximately 20°.

The cutting facets may have many different forms and orientations. Thus, when integrating the fiber exits at these facets as in a needle according to the invention, various fiber exit geometries become possible. Although tuning of the cutting facets is strongly determined by the cutting properties required for the needle they allow still a lot of freedom in designing fiber exit geometries. Furthermore, thanks to the orientation of the cutting facet, and thus the fiber end, no protrusion of the fiber end is needed.

According to the invention, the needle comprises a pair of side facets symmetrically arranged with respect to the centre axis of the needle, and the needle comprises a pair of front facets symmetrically arranged with respect to the centre axis of the needle.

'Side facets' may be formed by cutting the shaft of the needle at its side in the area of the bevel. It should be noted that the angle from the main axis to the side may be a pointed angle and the surface of the facet may also be inclined above or down with respect to the needle.

'Front facets' may be formed by cutting the pointed tip of the bevel at a blunt angle to the main axis. Therefore, usually two additional surfaces are formed with an edge between them, wherein said edge is located in the middle of the tip. Each of the two facet surfaces is inclined to the side and may also be inclined upwards or downwards.

The needle according to another embodiment of the invention may comprise a pair of fibers, wherein the end surfaces of the fibers may be located in different facets. The fibers may be located in facets located on different sides of the needle or both fibers may be located at one side of the needle, wherein the end surface of one of the fibers may be located at a side facet and the other one of the fibers may be located at a front facet, respectively.

It should be noted that the end surface of a fiber will have a circular shape or an oval shape, in case of a substantially circular cross section of the fiber. Depending on the angle at which the fiber will end at the corresponding facet, the shape of the end surface of the fiber will be effected and therefore also the direction of the emitted or received light.

According to a preferred embodiment of the invention, the needle further comprises a connector part, wherein a proximal end section of the fiber is located in the connector part for connection with a fiber cable located between the needle and a console including a light source and a light detector. With this, the proximal fiber end is rigidly mounted on the needle. In other words, there will be no fiber section leading out of the holder part of the needle.

Advantageously, the manufacturing, sterilization and handling of the needle is significantly simplified, the separate fiber cable can be made more robust since this part may be reused, and the workflow for setting up the equipment becomes easier since this separate fiber cable can be setup beforehand, i.e. the needle need not to be unpacked from its sterile enviroment.

According to a preferred embodiment of the invention, the needle with fibers might be used in a system for optical tissue inspection, wherein the system further comprises a light source connected with one of the fibers of the needle, a light detector connected with another one of the fibers of the needle, wherein light coming from the light source and being emitted from the end surface of the one of the fibers can be detected by the light detector when entering the other one of the fibers, a processing unit for processing the data from the light detector, and a monitor for visualization of the processed data. Between the needle and the light source and/or the light detector, a separate fiber cable may be provided.

It has to be noted that embodiments of the invention are described with reference to different subject matters. In particular, some aspects are described with reference to devices or systems whereas other aspects are described with reference to method steps. However, a person skilled in the art will gather from the above and the following description that, unless other notified, in addition to any combination of features belonging to one type of subject matter also any combination between features relating to different subject matters is considered to be disclosed with this application.

The aspects defined above and further aspects, features and advantages of the present invention can also be derived from the examples of embodiments to be described hereinafter and are explained with reference to examples of embodiments. The invention will be described in more detail hereinafter with reference to examples of embodiments but to which the invention is not limited.

### BRIEF DESCRIPTION OF THE DRAWINGS

Figure 1 shows a side view and a top view of a needle as well as schematically a system according to one embodiment of the invention.
Figure 2 illustrates in a schematic over view and in a detailed view, the aspect of connecting a separate fiber cable with the needle according to the invention.
Figures 3a and 3b show a top view and a side view of the tip part of a needle according to an embodiment of the invention.
Figures 4a and 4b show a top view and a side view of the tip part including fiber ends at the side facets.
Figures 5a and 5b show a top view and a side view of a tip part including fiber ends at one side of the needle.
Figure 6 shows a flow chart illustrating different steps of a method for producing a needle according to the invention.

The illustration in the drawings is schematically only and not to scale. It is noted in different figures, similar elements are provided with the same reference signs.

### DETAILED DESCRIPTION OF EXEMPLARY EMBODIMENTS

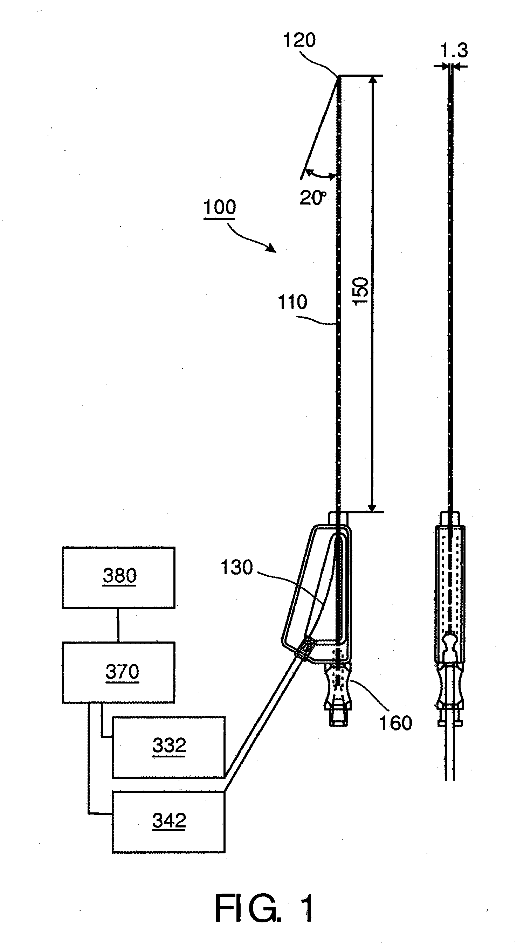

As illustrated in figure 1, a needle 100 according to one embodiment of the invention, comprises a shaft 110, a bevel 120 at the tip portion of the shaft, a fiber 130, and a holder part 160. According to this exemplary embodiment, the shaft has a length of 150 mm and a diameter of 1.3 mm. Further, the bevel encloses an angle with the shaft axis of 20°.

In this embodiment, the fiber 130 which runs from the distal end, i.e. the bevel 120, through the shaft 110 to the holder part 160, passes through an opening of the holder part 160 out of the needle.

The end surfaces of the fibers may be arranged at a distance to each other. Preferably, the distance between the fiber ends is greater than the diameter of the shaft. For example, the distance may be more than 1.1 times greater than the diameter. Particularly, the distance may be more than 1.25 times greater than the diameter. Preferably, the distance may be more than 1.5 times greater than the diameter. In other words, the distance between the fiber ends at the tip part of the needle should be as great as possible.

It is noted that the distances are measured from the central axis of one of the fibers to the central axis of the other one of the fibers. Furthermore, it is noted that the dimensions mentioned above, are exemplary and not intended to limit the scope of the invention. With the mentioned dimensions, it is intended to provide an order of magnitude and relations for a needle for tissue inspection based on optical spectroscopy.

As example depending on the intended use of the needle, the outer diameter of the needle might be 2.108 mm for a brain biopsy needle, between 1.27 mm and 2.108 mm for a common biopsy needle or a neuro puncture needle, between 0.711 mm and 2.108 mm for a fine aspiration needle, between 0.711 mm and 1.473 mm for an epidural needle, and might be 2.108 mm or smaller for a needle electrode.

The shaft 110 may comprise two hollow cylinders having different diameters. The use of said two cylinders enables an easy way to incorporate the fiber into the needle. Furthermore, the use of a separate tip part may enable a large freedom in assembling the illumination and collection fiber end geometries without having to make a complex mount that extends over the entire needle length.

The tip portion of the needle as well as the shaft of the needle might be made of metal, wherein the metal might be MRI compatible such as Titanium. The needle tip might also be made of a ceramic material. This has the advantage of being mouldable in various shapes while still allowing for a sharp and robust needle tip.

The holder part 160 is provided with a channel to guide the fibers from the tip of the needle, being mounted in the shaft, towards the outside world. This holder part has as additional functionality to provide strength around the fiber to prevent large bending angles of the fibers at the proximal end of the needle. The holder part might be made by plastic injection moulding.

Furthermore, in figure 1 are schematically illustrated the elements of a system according to the invention. The system includes the needle 100, a light source 332, a light detector 342, a processing unit 370 and a monitor 380. The processing unit 370 is capable of controlling the light source 332 to emit light into the fiber 130 such that light will be emitted through the distal end surface of the fiber 130 at the top of the bevel 120 into surrounding tissue. Depending on what kind of tissue is in front of the bevel, more or less of the emitted light will be reflected in the direction of the bottom of the bevel, to be received by another fiber. Through said other fiber, the light will be led to the light detector 342, which detector is adapted to transform the light into electrical signals. These electrical signals will be send by, for example, wire to the processing unit 370. The processing unit will process the data corresponding to the electrical signals, so that the processed data might be visualized on a monitor 380. Based on said visualized data, it might be possible to diagnose whether or not a tissue is cancerous.

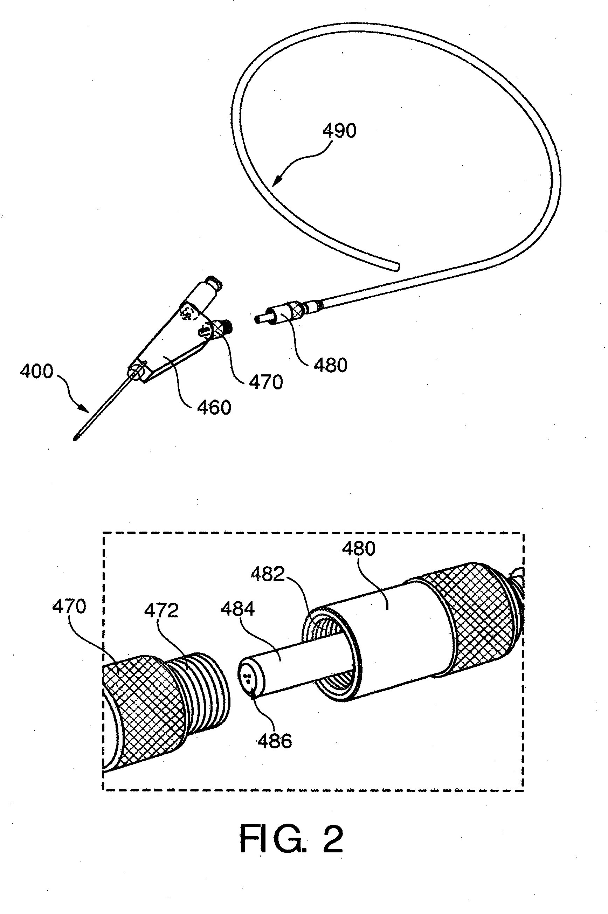

Figure 2 illustrates a connection of a fiber cable 490 with the holder part 460 of the needle 400. To make the connection to a console, a separate fiber connector cable 490 may be used or a fiber cable that is pre-connected to the console. This separate fiber cable need not be sterilized. In case this is required a plastic sterile sleeve can be put around it as is already commonly used in medical equipment.

To couple the needle 400 with the fiber cable 490, there is provided a connector part 470 at the needle 400, and a correspondingly formed connector part 480 at the fiber cable 490. As depict in the detail view in figure 2, the connector part 470 which is mounted at the holder part 460, includes a smaller end portion 472 and a recess (not shown) in its front surface. On the other hand, the connector part 480 at the fiber cable 490 includes in axial direction, a hollow portion 482, a pin like element 484 and a front surface 486.

During connection of the connector parts 470, 480, the pin like element 484 will be accommodated in the recess of the connector part 470, and the smaller end portion 472 will engage in the hollow portion 482 of the counter connector part 480. Flush with the blunt front surface 486 of the connector part 480, there is positioned at least one fiber end. The corresponding end(s) of the fiber(s) in the needle are located in the end surface of the recess of the connector part 470. Therefore, an appropriate and reliable connection between the two connector parts and thus between the fibers of the needle and the fiber cable is easily realized.

It is noted that a rigid connector 470 on the needle can either connect one fiber in the needle or a multiple number. In the example of figure 2, there are three fiber ends visible at the end surface 486 of the pin like element 484 of the connector part 480 at the end of the fiber cable 490.

There may be only one connector present on the needle, also multiple connectors are envisioned. The connector may also provide connection to other signals such as electrical signals.

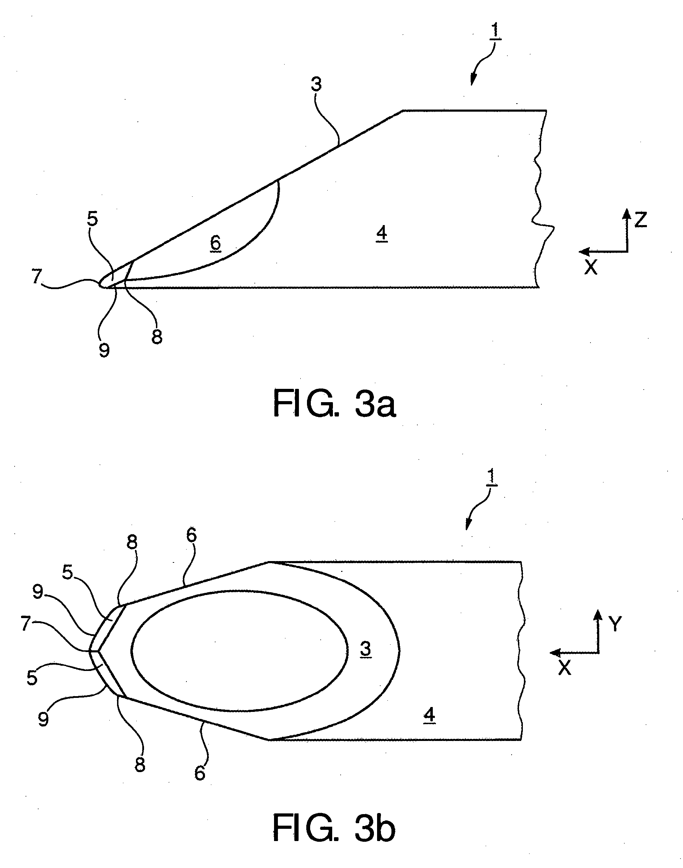

In the side view of figure 3a and in the top view of figure 3b, there is illustrated a tip part of a needle according to an embodiment of the invention. The tip part might be made of an appropriate metal material or alloy or might be made of a ceramic material.

The tip part 1 includes a shaft portion 4, a bevel 3 with a pointed tip, side facets 6, and front facets 5. Between the the two front facets 5, there is formed an front cutting edge 7. The lower edge between the surface of the shaft portion 4 and a front facet 5 is formed as lower cutting edge 9. From a front facet 5 to the side, i.e. between a front facet 5 and a side facet 6, there is formed a side cutting edge 8.

The cutting facets may have many different forms and orientations. As a result when integrating the fiber exits at these locations various fiber exit geometries become possible. Although tuning of the cutting facets is strongly determined by the cutting properties required for the needle they allow still a lot of freedom in designing fiber exit geometries.

In the illustrated example, each side facet 6 is a plane surface extending parallel to the z-axis and inclined with a small angle to the x-axis. On the other hand, each front facet 5 is also a plane surface, but is inclined with an angle to the x-axis and also to the z-axis.

It may be envisaged, for example, that the orientation of a side facet 6 may have another angle to the x-axis and may also have an angle to the z-axis. A front facet may have a more or less acute angle to the x-axis and may have also another angle to the z-axis. Even an orientation of a front facet parallel to the z-axis may be possible. As a further alternative, the front facets 5 and/or the side facets 6 may face downwardly.

Advantageously, the cutting facets may be covered by a coating that not only influences the cutting properties but also improve the optical properties. For instance the coating may serve as a diffuser for light emitted from a fiber end.

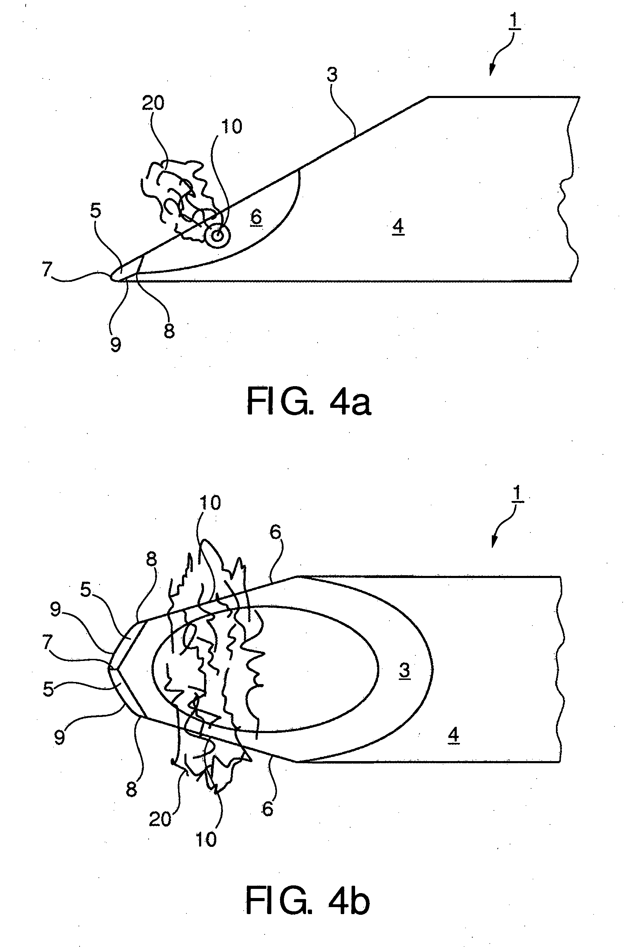

In figure 4a and 4b an example is shown where the fiber exits 10 are integrated in the cutting facets 6 on both sides. As a result the fiber ends look more outwards resulting in a larger probing volume 20 that also probe partly aside the needle.

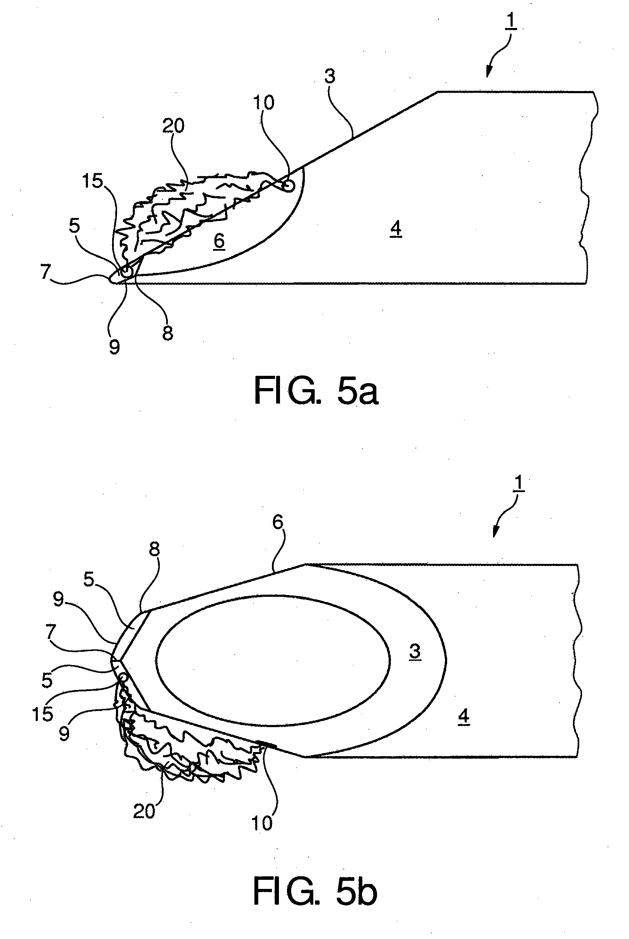

In the embodiment shown in figure 5a and 5b the fiber exits 10 and 15 are integrated in the cutting edges 6 and 5, respectively. The volume probed by the light indicated by 20 looks now more to one side of the needle.

As can be seen by comparing figure 4a and 5a, the location of the fiber end 10 may be on different positions on the surface of a facet. As discussed above, it is preferred to have a distance between two fiber ends, which should be as great as possible. Therefore, in the example of figure 4, the fiber end 10 may be in the middle of the facet 6. In contrast to this in figure 5a, since the fiber ends 10 and 15 are both located at one side of the needle, the fiber end 10 is located near the upper or proximal end of the surface of the side facet 6, to maximize the distance between the fiber ends 10 and 15.

Apart from the fiber exits integrated in at least one of the facets, fibers exits may also be integrated in other parts such as the bevel 3 and/or the shaft portion 4.

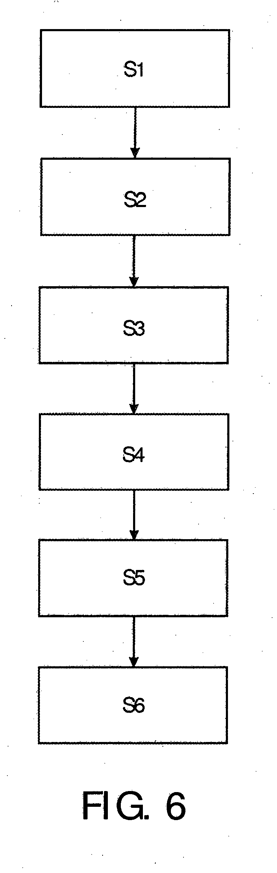

Figure 6 is a flow chart, showing the steps of a method for producing a needle according to the invention. It will be understood, that the steps described with respect to the method, are major steps, wherein these major steps might be differentiated or divided into several sub steps. Furthermore, there might be also sub steps between these major steps. Therefore, a sub step is only mentioned, if said step is important for the understanding of the principles of the method according to the invention.

Step S 1 of the method according to the invention, is the manufacturing of the tip part, wherein this manufacturing includes forming of at least one channel in axial direction and forming facets.

Step S2 is the positioning of an end section of at least one fiber in a respective channel, wherein said positioning might include the fixing of the at least one fiber by, for example, gluing.

Step S3 is the connecting of the distal end of the shaft with the tip part.

Step S4 is the connecting of the proximal end of the shaft with the holder part, wherein the at least one fiber will be passed through the shaft and into the holder part.

Step S5 is the fixating of the proximal end of the at least one fiber by means of a connector part mounted at the holder part.

Step S6 of the method according to the invention, is the polishing of the tip part and especially of the end surface of the at least one fiber such that the end surface of the at least one fiber is flush with the surface of the tip part.

It is noted that the fixing of the at least one fiber in the respective channel might be provided by gluing, and the connecting of the shaft with the tip part and/or the holder part, might be provided by welding or by gluing.

The needles according to the invention can be used in minimally invasive needle interventions such as low-back pain interventions or taking biopsies in the field of cancer diagnosis or in case where tissue characterization around the needle is required.

In the following, exemplary needles according to the invention will be described with respect to their outer diameter, their insertion length, and their preferred use.

A biopsy needle might have an outer diameter of 1.27 mm up to 2.108 mm, might be inserted into tissue with 100 mm to 150 mm of its length, and might be used in soft tissue core biopsies in the neck, the head, the breast, the prostate, and the liver.

A fine aspiration needle of soft tissue might have an outer diameter between 0.711 mm and 2.108 mm, might be inserted into soft tissue with 100 mm to 150 mm of its length, and might be used for aspiration of soft tissue.

A brain biopsy needle might have an outer diameter of 2.108 mm, might be inserted into tissue with 150 mm up to 250 mm of its length, and might be used for diagnostic brain biopsies.

A neuro puncture needle might have an outer diameter of 1.27 mm up to 2.108 mm, might be inserted into tissue with 150 mm to 200 mm of its length, wherein such needles allow a non-traumatic approach to lesions in the brain.

An epidural needle might have an outer diameter between 0.711 mm and 1.473 mm, might be inserted into tissue with a length of up to 150 mm, and might be used for treatments in the spinal cord area such as steroid injections in the epidural space.

Finally, a needle electrode might have an outer diameter of 2.108 mm and smaller, might be inserted into tissue up to 250 mm of its length, and might be used for radio frequency ablation for instance of tumors.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements, and the indefinite article "a" or "an" does not exclude a plurality. A single unit may fulfill the functions of several items recited in the claims. The mere fact that certain measures are recited in mutually different dependent claims does not indicate that a combination of these measured cannot be used to advantage. Any reference signs in the claims should not be construed as limiting the scope.

### LIST OF REFERENCE SIGNS

- 100, 400: needle
- 4, 110: shaft
- 3, 120: bevel
- 10, 15, 130: fiber
- 1: tip part
- 5: front facet
- 6: side facet
- 7: front cutting edge
- 8: side cutting edge
- 9: lower cutting edge
- 20: probing volume
- 160, 460: holder part
- 332: light source
- 342: light detector
- 370: processing unit
- 380: monitor
- 470, 480: connector part
- 472: smaller end portion
- 482: hollow portion
- 484: pin like element
- 486: front surface
- 490: fiber cable

## Claims

1. A needle (100, 400) comprising:
- a tip part (1) having a main axis, and
- fibers (10, 15, 130) capable of transmitting light,
- wherein the tip part comprises a bevel (3, 120), cutting facets (5, 6) which are plane surfaces, wherein the cutting facets include a pair of side facets (6) being formed with a pointed angle from the main axis to a side of the tip part and being symmetrically arranged with respect to the main axis, and at least one channel for accomodating a respective fiber,
- wherein an end section of said fiber is located in the channel, and wherein end surfaces of said fibers are located in different facets, **characterised in that**:
the cutting facets further include a pair of front facets (5) being formed with a blunt angle to the main axis and being symmetrically arranged with respect to the main axis.

2. The needle of claim 1, wherein the needle comprises a pair of fibers, wherein the end surfaces of the pair of fibers are located at facets (5, 6) at different sides of the needle.

3. The needle of claim 1, wherein the needle comprises a pair of fibers (10, 15, 130), wherein both fibers are located at one side of the needle (100, 400) and wherein the end surface of one of the fibers is located in the side facet (6) and the other one of the fibers is located in the front facet (5), respectively.

4. The needle of claim 1, wherein a proximal end section of the fiber (10, 15, 130) is located in a connector part (470) for connection with a fiber cable (490) located between the needle (100, 400) and a console including a light source (332) and a light detector (342).

5. A system for optical tissue inspection, the system comprising
- a needle (100, 400) according to claim 1,
- a light source (332) connected with one of the fibers (10, 15, 130) of the needle,
- a light detector (342) connected with another one of the fibers (10, 15, 130) of the needle, wherein light coming from the light source and being emitted from the end surface of the one of the fibers can be detected by the light detector when entering one of the fibers,
- a processing unit (370) for processing the data from the light detector, and
- a monitor (380) for visualization of the processed data.

6. The system of claim 5, further comprising
- a fiber cable (490) coupling the proximal end of a fiber (10, 15, 130) of the needle (100, 400) with the light source (332) or light detector (342).

## Patentansprüche

1. Nadel (100, 400), die Folgendes umfasst:
- einen Spitzenteil (1) mit einer Hauptachse, und
- Fasern (10, 15, 130), die in der Lage sind, Licht zu übertragen,
- wobei der Spitzenteil umfasst: eine Schrägkante (3, 120), Schneidfacetten (5, 6), die ebene Flächen sind, wobei die Schneidfacetten ein Paar Seitenfacetten (6) umfassen, die mit einem spitzen Winkel ausgehend von der Hauptachse zu einer Seite des Spitzenteils hin gebildet sind und symmetrisch in Bezug auf die Hauptachse angeordnet sind, und mindestens einen Kanal zur Aufnahme einer jeweiligen Faser,
- wobei sich ein Endabschnitt der genannten Faser in dem Kanal befindet, und wobei sich Endflächen der genannten Fasern in unterschiedlichen Facetten befinden, **dadurch gekennzeichnet, dass** die Schneidfacetten weiterhin ein Paar vordere Facetten (5) umfassen, die mit einem stumpfen Winkel zu der Hauptachse gebildet sind und symmetrisch in Bezug auf die Hauptachse angeordnet sind.

2. Nadel nach Anspruch 1, wobei die Nadel ein Faserpaar umfasst, wobei sich die Endflächen des Faserpaars bei Facetten (5, 6) auf unterschiedlichen Seiten der Nadel befinden.

3. Nadel nach Anspruch 1, wobei die Nadel ein Faserpaar (10, 15, 130) umfasst, wobei sich beide Fasern auf einer Seite der Nadel (100, 400) befinden und wobei sich die Endfläche von einer der Fasern in der Seitenfacette (6) befindet und die der anderen Faser sich in der vorderen Facette (5) befindet.

4. Nadel nach Anspruch 1, wobei sich ein proximaler Endabschnitt der Faser (10, 15, 130) in einem Konnektorteil (470) zur Verbindung mit einem Faserkabel (490) befindet, das zwischen der Nadel (100, 400) und einer Konsole mit einer Lichtquelle (332) und einem Lichtdetektor (342) angeordnet ist.

5. System zur optischen Gewebeuntersuchung, wobei das System Folgendes umfasst:
- eine Nadel (100, 400) nach Anspruch 1,
- eine Lichtquelle (332), die mit einer der Fasern (10, 15, 130) der Nadel verbunden ist,
- einen Lichtdetektor (342), der mit einer anderen der Fasern (10, 15, 130) der Nadel verbunden ist, wobei das aus der Lichtquelle stammende und aus der Endfläche von der einen der Fasern emittierte Licht durch den Lichtdetektor detektiert werden kann, wenn es in eine der Fasern eintritt,
- eine Verarbeitungseinheit (370) zum Verarbeiten der Daten aus dem Lichtdetektor, und
- einen Monitor (380) zum Visualisieren der verarbeiteten Daten.

6. System nach Anspruch 5, das weiterhin Folgendes umfasst:
- ein Faserkabel (490), das das proximale Ende einer Faser (10, 15, 130) der Nadel (100, 400) mit der Lichtquelle (332) oder Lichtdetektor (342) koppelt.

## Revendications

1. Aiguille (100, 400), comprenant:
- une partie d'embout (1) possédant un axe principal, et
- des fibres (10, 15, 130) capables de transmettre de la lumière,
- dans laquelle la partie d'embout comprend un biseau (3, 120), des facettes de coupe (5, 6) qui sont des surfaces planes, dans laquelle les facettes de coupe comprennent une paire de facettes latérales (6) formées avec un angle pointu de l'axe principal à un côté de la partie d'embout et agencées symétriquement par rapport à l'axe principal, et au moins un canal pour loger une fibre respective,
- dans laquelle une section d'extrémité de ladite fibre est située dans le canal, et dans laquelle des surfaces d'extrémité desdites fibres sont situées dans des facettes différentes, **caractérisée en ce que** les facettes de coupe comprennent en outre une paire de facettes avant (5) formées avec un angle obtus par rapport à l'axe principal et agencées symétriquement par rapport à l'axe principal.

2. Aiguille selon la revendication 1, dans laquelle l'aiguille comprend une paire de fibres, dans laquelle les surfaces d'extrémité de la paire de fibres sont situées sur des facettes (5, 6) sur des côtés différents de l'aiguille.

3. Aiguille selon la revendication 1, dans laquelle l'aiguille comprend une paire de fibres (10, 15, 130), dans laquelle les deux fibres sont situées à une extrémité de l'aiguille (100, 400) et dans laquelle la surface d'extrémité d'une des fibres est située dans la facette latérale (6) et l'autre des fibres est située dans la facette avant (5), respectivement.

4. Aiguille selon la revendication 1, dans laquelle une section d'extrémité proximale de la fibre (10, 15, 130) est située dans une partie de connexion (470) pour la connexion à un câble à fibre (490) situé entre l'aiguille (100, 400) et une console comprenant une source lumineuse (332) et un détecteur de lumière (342).

5. Système pour l'inspection optique de tissu, le système comprenant :
- une aiguille (100, 400) selon la revendication 1,
- une source lumineuse (332) connectée à une des fibres (10, 15, 130) de l'aiguille,
- un détecteur de lumière (342) connecté à une autre des fibres (10, 15, 130) de l'aiguille, dans lequel de la lumière provenant de la source lumineuse et émise à partir de la surface d'extrémité de l'une des fibres peut être détectée par le détecteur de lumière lors de l'entrée dans une des fibres,
- une unité de traitement (370) pour traiter les données à partir du détecteur de lumière, et
- un moniteur (380) pour la visualisation des données traitées.

6. Système selon la revendication 5, comprenant en outre :
- un câble à fibre (490) couplant l'extrémité proximale d'une fibre (10, 15, 130) de l'aiguille (100, 400) avec la source lumineuse (332) ou le détecteur de lumière (342).
